(19) Europäisches Patentamt
European Patent Office
Office européen des brevets

(11) Veröffentlichungsnummer : **0 245 792 B1**

# (12) EUROPÄISCHE PATENTSCHRIFT

(45) Veröffentlichungstag der Patentschrift : **12.06.91 Patentblatt 91/24**

(51) Int. Cl.$^5$ : **C07C 67/08**

(21) Anmeldenummer : **87106694.0**

(22) Anmeldetag : **08.05.87**

## (54) Verfahren zur Herstellung von Carbonsäureestern.

(30) Priorität : **14.05.86 DE 3616137**

(43) Veröffentlichungstag der Anmeldung : **19.11.87 Patentblatt 87/47**

(45) Bekanntmachung des Hinweises auf die Patenterteilung : **12.06.91 Patentblatt 91/24**

(84) Benannte Vertragsstaaten : **BE CH DE FR GB IT LI NL**

(56) Entgegenhaltungen : **US-A- 2 622 071**

(73) Patentinhaber : **BAYER AG**
**W-5090 Leverkusen 1 Bayerwerk (DE)**

(72) Erfinder : **Meier, Helmut-Martin, Dr.**
**Am Obersthof 3**
**W-4030 Ratingen 6 (DE)**
Erfinder : **Kircher, Klaus, Dr.**
**Alfred-Kubin-Strasse 3**
**W-5090 Leverkusen (DE)**
Erfinder : **Berg, Klaus, Dr.**
**Hansastrasse 124**
**W-4150 Krefeld (DE)**

EP 0 245 792 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist die Verwendung von oxetangruppenhaltigen neutralen Phosphiten zur Herstellung von Carbonsäuraestern aus Carbonsäuren und Alkoholen.

Es ist bereits bekannt, Glycerin und Fettsäuren in Gegenwart von Metalloxidan oder Metallen wie Zinkoxid oder Zinn nach DRP 403 644 umzusetzen. Nach Houben-Weyl, "Methoden der Organischen Chemie", Sauerstoffverbindungen III, Band VIII, 1952, Seiten 516 ff, insbesondere Seite 517 ist es auch üblich, Mineralsäuren wie Salzsäure, Schwefelsäure, Phosphorsäure oder organische Säuren wie aromatische Sulfonsäure oder saure Salze als Veresterungskatalysatoren einzusetzen. Im US-Patent 4 526 725 wird die Veresterung von Carbonsäuren oder Carbonsäureanhydriden mit Alkoholen in Gegenwart einar mit Bishydroxyethyl-amin modifizierten Alkoxy-Titanverbindung beschrieben.

α-Hydroxyalkylphosphite sind als Antivergilbungsreagentien in Überzugsmitteln nach DE-PS 3 422 668 bekannt.

Ferner gibt es nach EP 0 158 501 ein Verfahren zur Herstellung von Polyestern in Gegenwart von Phosphoniten. Schließlich ist noch ein Verfahren in DOS 3 516 776 zur Herstellung von Kolophonium-Pentaerythritestern in Gegenwart von Phosphinsäure beschrieben.

Die in DRP 403 644 und US 4 526 725 beschriebenen Methoden zur Veresterung liefern ein Endprodukt mit hoher OH-Zahl, das wegen seines Metallgehalts nicht als Additiv in einem durchsichtigen Kunststoff eingesetzt werden kann, da entsprechende Produkte eine Trübung aufweisen.

Nach den in Houben-Weyl, loc. cit. vorgeschlagenen Verfahren erhält man Ester, die im Endprodukt eine zu hohe Säurezahl aufweisen und zu störenden Folgereaktionen bei der Verwendung als Additiv in gewissen Kunststoffen führen können. Außerdem ist die Eigenfarbe, der mit diesen Veresterungskatalysatoren erhaltenen Ester zu dunkel für eine Anwendung als Additiv in hellen oder farblosen Kunststoffteilen.

Gemäß US-Patent 2 622 071 und US-Patent 2 676 158 werden phenolische OH-Gruppen unter Verwendung von aliphatischen bzw. aromatischen Phosphiten mit Carbonsäuren verestert.

Die in DE-P 3 422 668, EP 0 158 501 und DOS 3 516 776 beschriebenen phosphorhaltigen Verbindungen sind, wie die Vergleichsbeispiele belegen, keine geeigneten Veresterungskatalysatoren, da sie die vorstehend beschriebenen Nachteile nicht vermeiden helfen.

Überraschenderweise wurde nun gefunden, daß man diese Nachteile vermeidet, wenn man die Veresterung in Gegenwart oxetangruppenhaltiger neutraler Phosphite durchführt.

Geeignete oxetangruppenhaltige neutrale Phosphite sind beispielsweise Verbindungen der formel (I)

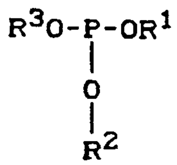

(I),

worin mindestens einer der Reste $R^1$, $R^2$ und $R^3$ der Rest eines oxetangruppenhaltigen Monoalkohols ist, während maximal 2 der Reste $R^1$, $R^2$ und $R^3$ Reste primärer, sekundärer oder tertiärer aliphatischer, cycloaliphatischer oder heterocyclischer, oxetangruppenfreier Monoalkohole oder oxetangruppenfreier Monohydroxyaryle sind.

Vorzugsweise geeignete oxetangruppenhaltige Monoalkohole sind solche der formel (II)

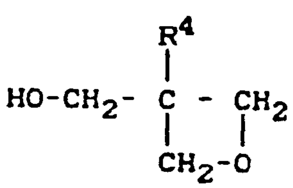

(II),

worin $R^4$, H, $C_1$-$C_{18}$-Alkyl, $C_3$-$C_{12}$-Cycloalkyl, gegebenenfalls alkylsubstituiertes $C_6$-$C_{14}$-Aryl, $C_7$-$C_{18}$-Aralkyl oder $-CH_2$-O-R, wobei R wiederum die für $R^4$ genannten Alkyl, Cycloalkyl, Aryl und Aralkyl sein kann.

Vorzugsweise ist $R^4$ ein $C_1$-$C_5$-Alkylrest.

Geeignete oxetangruppenhaltige Monoalkohole der Formel (II) sind beispielsweisee 3-Hydroxymethyloxetan, 3-Methyl-3-hydroxymethyloxetan, 3-Ethyl-3-hydroxymethyloxetan, 3-pentyl-3-hydroxymethyloxetan, 3-Hexadecyl-3-hydroxymethyloxetan, 3-Octadecyl-3-hydroxymethyloxetan, 3-Cyclohexyl-3-hydroxymethyloxetan, 3-Phenyl-3-hydroxymethyloxetan, 3-p-Tolyl-3-hydroxymethyloxetan, 3-Benzyl-3-hydroxymethyloxetan, 3-Methoxymethyl-3-hydroxymethyloxetan, 3-Ethoxymethyloxetan, 3-Octadecylmethyl-3-hydroxymethyloxetan, 3-Phenoxymethyl-3-hydroxymethyloxetan, 3-p-Tolyloxymethyl-3-hydroxymethyloxetan und 3-Benzyloxymethyl-3-hydroxymethyloxetan.

Weitere geeignete oxetangruppenhaltige Monoalkohole, die nicht der Formel (II) entsprechen, sind beispielsweisee 3-Phenoxy-3-hydroxymethyloxetan, 3-p-Chlorphenoxy-3-hydroxymethyloxetan, 3-p-tert.-Butylphenoxy-3-hydroxymethyloxetan, 3-Acetyloxymethyl-3-hydroxymethyloxetan und 3-Stearoyloxymethyl-3-hydroxymethyloxetan.

Als oxetangruppenfreie Monoalkohole und Monohydroxyaryle zur Herstellung der Phosphorigsäureester der Formel (I) sind beispielsweise primäre, aliphatische, oxetangruppenfreie $C_1$-$C_{18}$-Monoalkohole, sekundäre, aliphatische, oxetangruppenfreie $C_3$-$C_{18}$-Monoalkohole und tertiäre, aliphatische, oxetangruppenfreie $C_4$-$C_{18}$-Monoalkohole geeignet ; ebenso geeignet sind beispielsweise sekundäre, cycloaliphatische, oxetangruppenfreie $C_3$-$C_{12}$-Monoalkohole, primäre, cycloaliphatische, oxetangruppenfreie $C_4$-$C_{12}$-Monoalkohole oder tertiäre, cycloaliphatische, oxetangruppenfreie $C_4$-$C_{12}$-Monoalkohole, geeignet sind beispielsweise auch primäre, sekundäre oder tertiäre heterocyclische oxetangruppenfreie $C_3$-$C_{12}$-Monoalkohole.

Geeignete oxetangruppenfreie Monohydroxyverbindungen sind beispielsweise Methylalkohol, Ethylalkohol, N-Propylalkohol, Isopropylalkohol, N-Butyl-alkohol, Isobutyl-alkohol, tert.-Butylalkohol, n-Hexylalkohol, 2-Ethyl-hexanol-1, Decylalkohol, Laurylalkohol, Octacecylalkohol, S-Ethylenthioglykol, 5-Dodecylthioglykol, Cyclohexylalkohol, 2-Methyl-cyclohexylalkohol, Cyclobutanol, Cyclopropanol, Benzylalkohol, α-Methylbenzylalkohol, Tetrahydrofurfurylalkohol, 5-Hydroxymethyl-5-ethyl-1,3-dioxan, Ethylenglykolmonobutylester, Diacetin, Trimethylolpropandiallylether, Phenol, 2,6-Diisobutyl-p-methylphenol, α-Naphthol, β-Naphthol und p-Chlorphenol.

Erfindungsgemäß verwendbare oxetangruppenhaltige Phosphorigsäureester der Formel (I) sind beispielsweise Tris-(2,2-dimethylenoxid-butyl)-phosphit, Bis-(2,2-dimethylenoxid-butyl)-phenyl-phosphit, 2,2-Dimethylenoxid-butyl-bis(phenyl)phosphit, Bis-(2,2-dimethylenoxid-butyl)-decyl-phosphit, Bis-(2,2-dimethylenoxid-butyl)-p-tolyl-phosphit, 2,2-Dimethylenoxid-butyl-bis(o-chlorphenyl)-phosphit, Bis-(2,2-dimethylenoxid-butyl)-benzyl-phosphit, Bis-(2,2-dimethylenoxid-butyl)-octadecyl-phosphit, Bis-(2,2-dimethylenoxid-butyl)-methyl-phosphit, Bis-(2,2-dimethylenoxid-butyl)-cyclohexyl-phosphit, 2,2-Dimethylenoxid-butyl-bis(decyl)-phosphit, Tris-(2,2-dimethylenoxid-propyl)-phosphit, Bis-(2,2-dimethylenoxid-propyl)-phenyl-phosphit, 2,2-Dimethylenoxid-propyl-bis-(phenyl)-phosphit, Tris-(2,2-dimethylenoxid-octadecyl)-phosphit, Tris-(2,2-dimethylenoxid-2-phenyl-ethyl)-phosphit, Bis-(2,2-dimethylonoxid-2-phenyl-ethyl)-phenyl-phosphit, Tris-(2,2-dimethylenoxid-2-p-tolyl-ethyl)-phosphit, Tris-(2,2-dimethylenoxid-3-phenyl-propyl)-phosphit, Tris-(2,2-dimethylenoxid-3-methoxypropyl)-phosphit, Tris-(2,2-dimethylonoxid-3-ethoxypropyl)-phosphit, Tris-(2,2-dimethylenoxid-3-butoxypropyl)-phosphit, Tris-(2,2-dimethylenoxid-3-octadecyloxy-propyl)-phosphit, Tris-(2,2-dimethylenoxid-3-phenoxy-propyl)-phosphit und Tris-(2,2-dimethylenoxid-3-benzyloxy-propyl)-phosphit.

Erfindungsgemäß verwendbare Phosphorigsäureester sind auch solche von oxetangruppenhaltigen Polyalkoholen, insbesondere von oxetangruppenhaltigen Dialkoholen.

Die Phosphorigsäureester von oxetangruppenhaltigen Dialkoholen entsprechen den folgenden Strukturformeln (IIIa) und (IIIb)

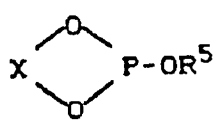

(IIIa)

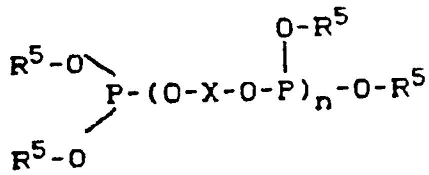

(IIIb)

worin X der Rest eines oxetangruppenhaltigen Dialkohols HO-X-OH mit X als oxetangruppenhaltigen organischen Rest mit beispielsweise 5 bis 20 C-Atomen, $R^5$ der Rest eines oxetangruppenfreien Monoalkohols oder eines oxetangruppenfreien Monohydroxyaryl und "n" eine ganze Zahl von 1 bis 10 einschließlich, vorzugsweise von 1 bis 3 einschließlich und insbesondere 1 oder 2 ist.

Geeignete oxetangruppenfreie Monoalkohole $R^5$-OH und oxetangruppenfreie Monohydroxyaryle $R^5$-OH sind die bereits für die Charakterisierung der Phosphorigsäureester (I) genannten.

Beispiele für oxetangruppenhaltige Dialkohole HO-X-OH sind

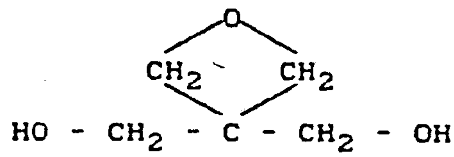 und

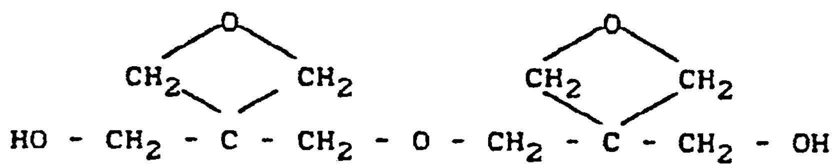 .

Phosphorigsäurester der Struktur (IIIa) beziehungsweise (IIIb) sind beispielsweise

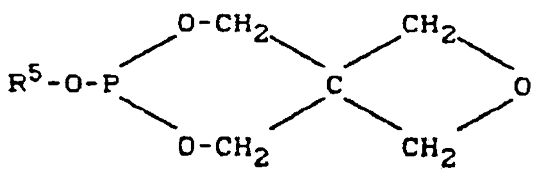

und

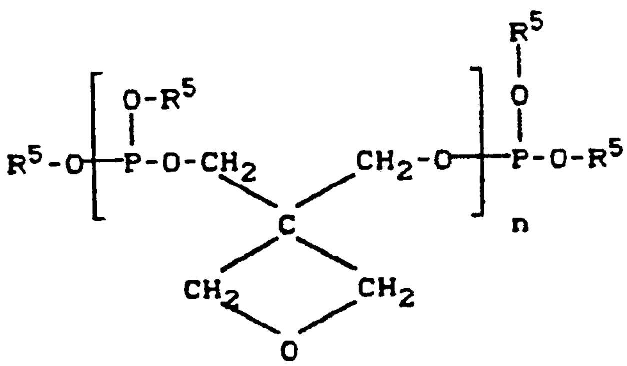

worin $R^5$ und "n" die für die Strukturfurmeln (IIIa) und (IIIb) genannte Bedeutung haben.

Erfindungsgemäß verwendbare Phosphorigsäureester sind auch solche von oxetangruppenhaltigen Monoalkoholen und aliphatischen, cycloaliphatischen oder heterocyclischen, oxetangruppenfreien Polyalkoholen, vorzugsweise Dialkoholen und/oder oxetangruppenfreien Polyhydroxyarylen, vorzugsweise Dihydroxyarylen.

Oxetangruppenfreie Polyalkohole sind beispielsweise solche mit 2 bis 20 C-Atomen wie etwa Ethylenglykol, 1,2-Propandiol, 2,2-Dimethylpropandiol, 2-Methylenpropandiol-1,3, 2,2-Dimethyl-4,4-dimethyl-cyclobutandiol-1,3, Glycerin, Trimethylolpropan, Pentaerythrit, Sorbit, Thiodiglykol, Diethylenglykol, Triethylenglykol, 2-Ethylpropandiol-1,3, 1,4-Butandiol, 1,4-Buten-2-diol, 1,6-n-Hexandiol, 4,4'Bishydroxycylcohexyl-2,2-propan, Cyclohexyl-1,4-dimethanol und 1,3-Hydroxy-2,2,4,4-tetramethylcyclobutan.

Ocetangruppenfreie Polyhydroxyaryle sind beispielsweise solche mit 6 bis 30 C-Atomen wie etwa Hydrochinon, Resercin, Brenzcatechin, Di-t-butyl-brenzcatechin, 4,4'-Dihydroxydiphenyl, Bis-(hydroxy-phenyl)-alkane wie beispielsweise $C_1$-$C_8$-Alkylen- bzw. $C_2$-$C_8$-Alkylidenbisphenole, Bis-(hydroxyphenyl)-cycloalkane wie beispielsweise $C_5$-$C_{15}$-Cycloalkylen- bzw. $C_5$-$C_{15}$-Cycloalkylidenbisphenole, $\alpha,\alpha'$-Bis-(hydroxyphenyl)-diisopropylbenzol sowie die entsprechenden kernalkylierten bzw. kernhalogenierten Verbindungen, beispielsweise Bis-(4-hydroxy-phenyl)-propan-2,2-(Bisphenol A), Bis-(4-hydroxy-3,5-dichlor-phenyl-propan-2,2 (Tetrachlorbisphenol A), Bis-(4-hydroxy-3,5-dibromphenyl)-propan-2,2 (Tetrabrombisphenol A), Bis-(4-hydroxy-3,5-dimethyl-phenyl)-propan-2,2 (Tetramethylbisphenol A), Bis-(4-hydroxy-phenyl)-cyclohexan-1,1 (Bisphenol Z) sowie $\alpha,\alpha'$-Bis-(4-hydroxyphenyl)-p-diisopropyl-benzol, Dihyronaphthaline, Dihydroxyanthracene, Chlorglucin, Pyrogallol, Bis-(2-hydroxy-3-tert.-butyl-phenyl)-methan, Bis(2-hydroxy-3-cyclohexylphenyl)-sulfid, Bis-(2-hydroxy-3-methylphenyl)-ether und Bis-(2-hydroxy-3-tert.-butyl-5-methylphenyl)-propan-2,2.

Oxetangruppenhaltige Phosphorigsäureester mit oxetangruppenfreien Polyalkoholen sind beispielsweise

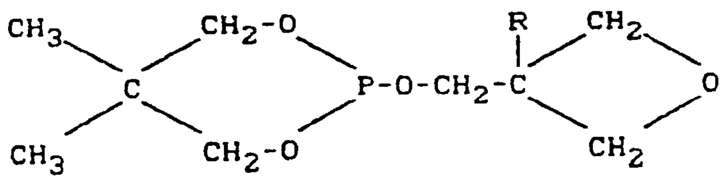

(mit R beispielsweise H oder $C_1$-$C_{18}$-Alkyl),

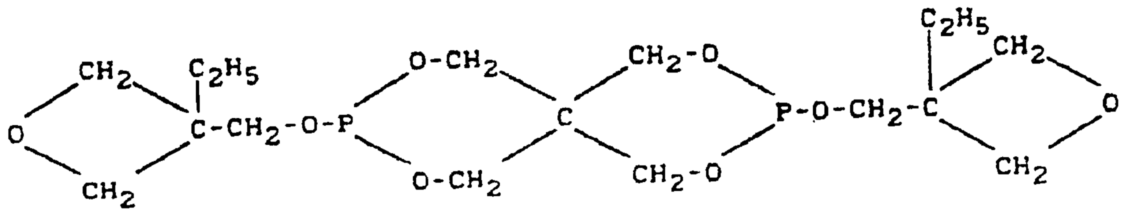

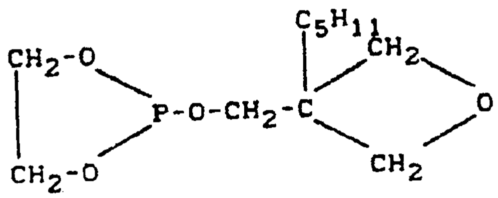

und

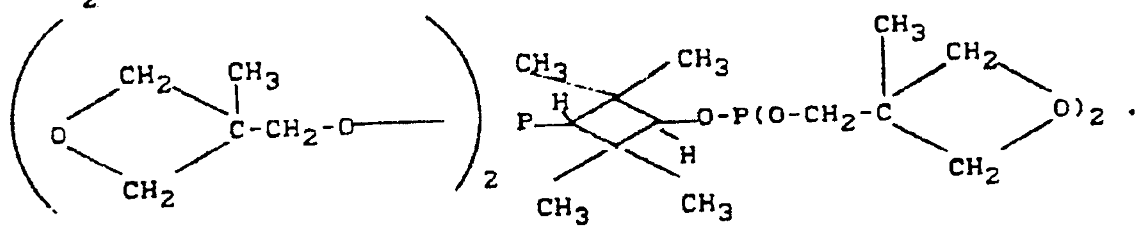

Oxetangruppenhaltige Phosphorigsäureester mit oxetangruppenfreien Polyhydroxylarylen sind beispielsweise

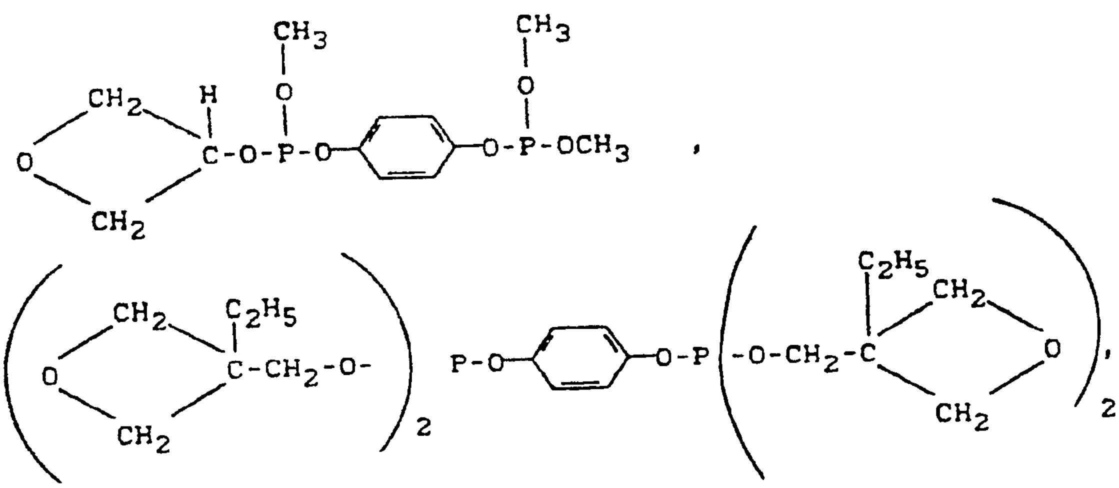

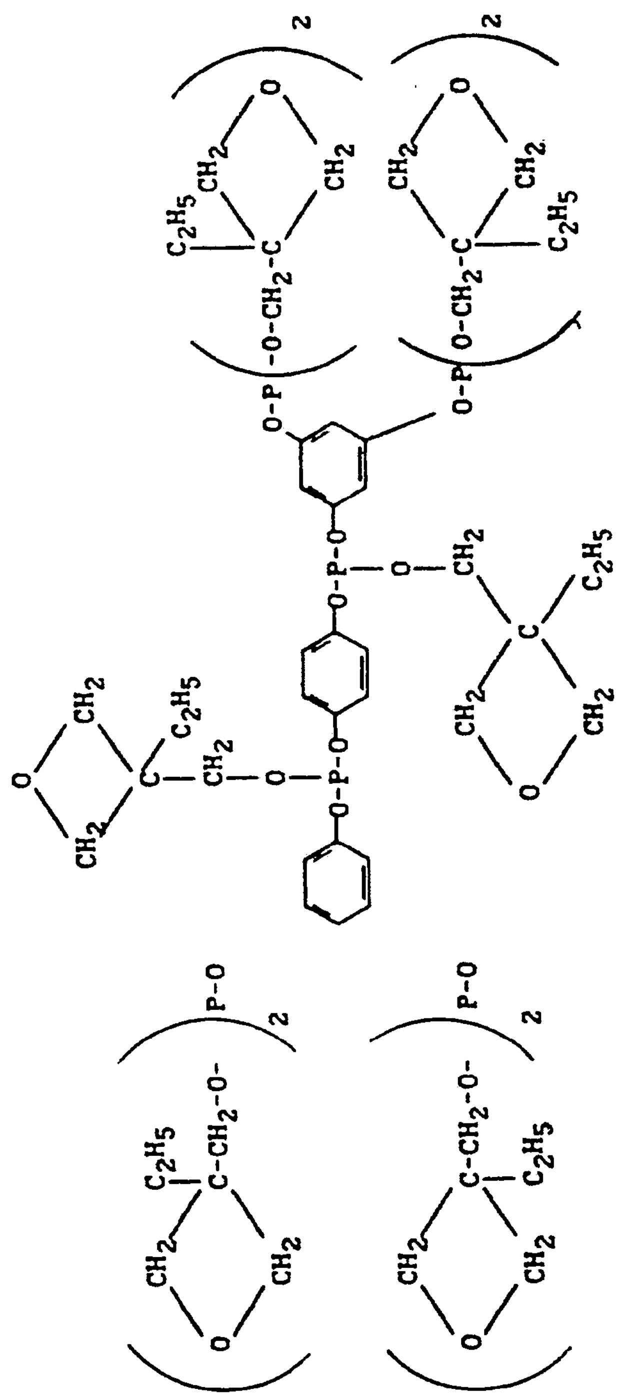

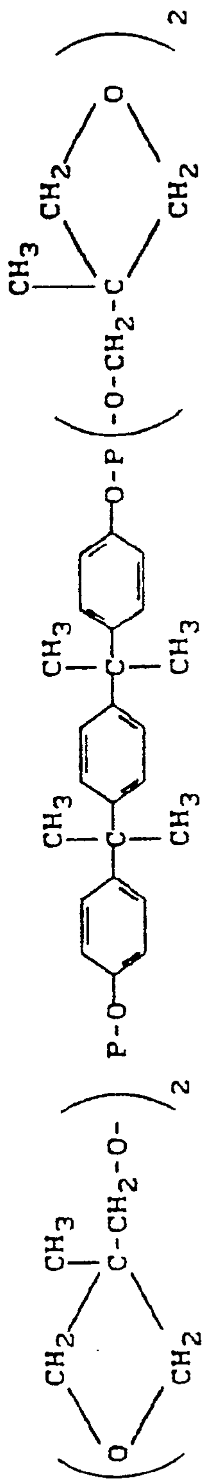

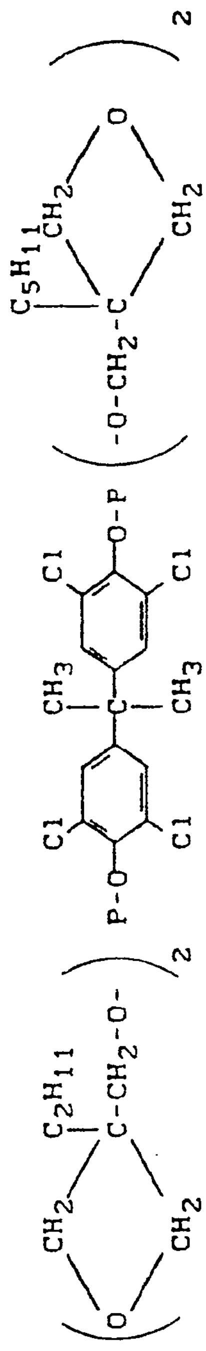

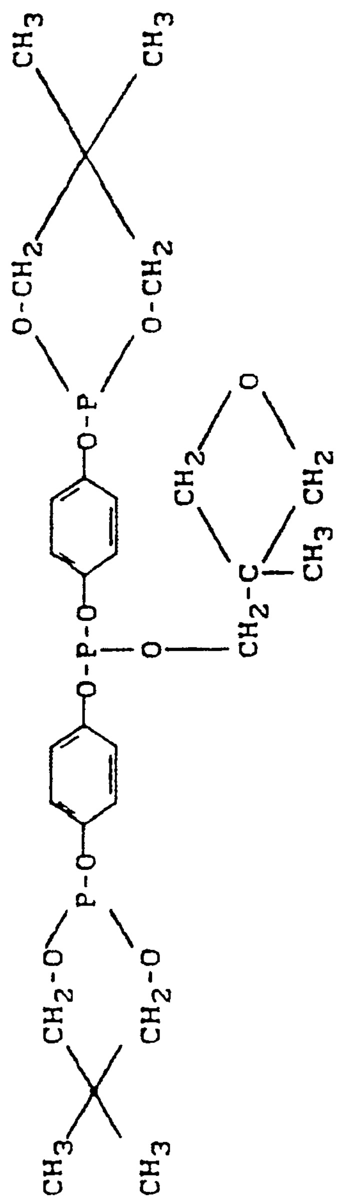

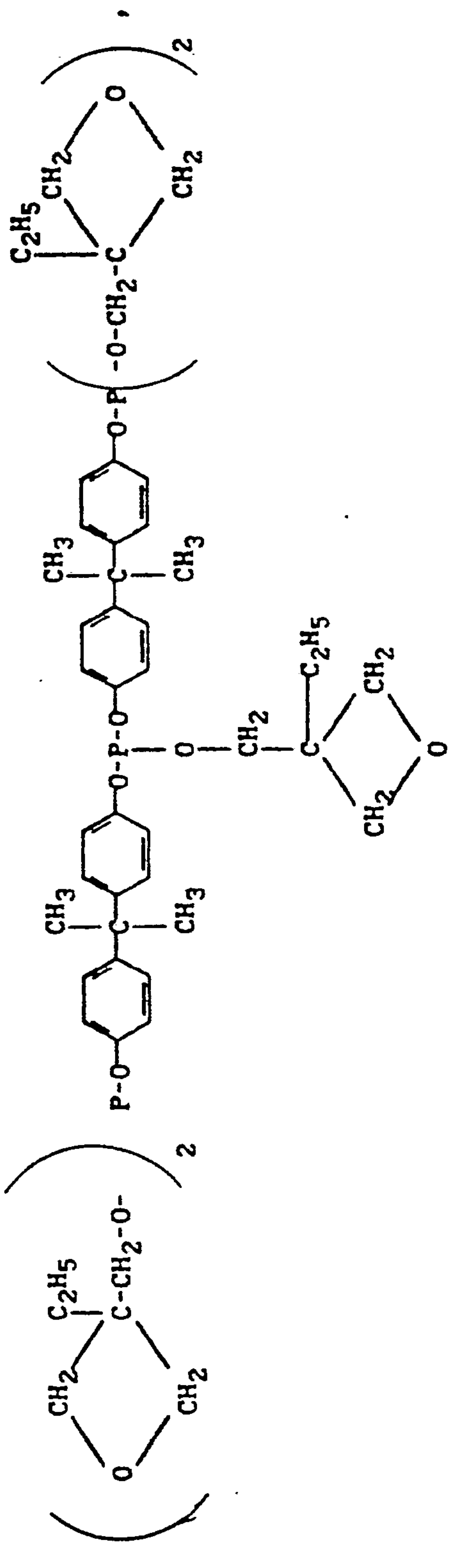

,

und

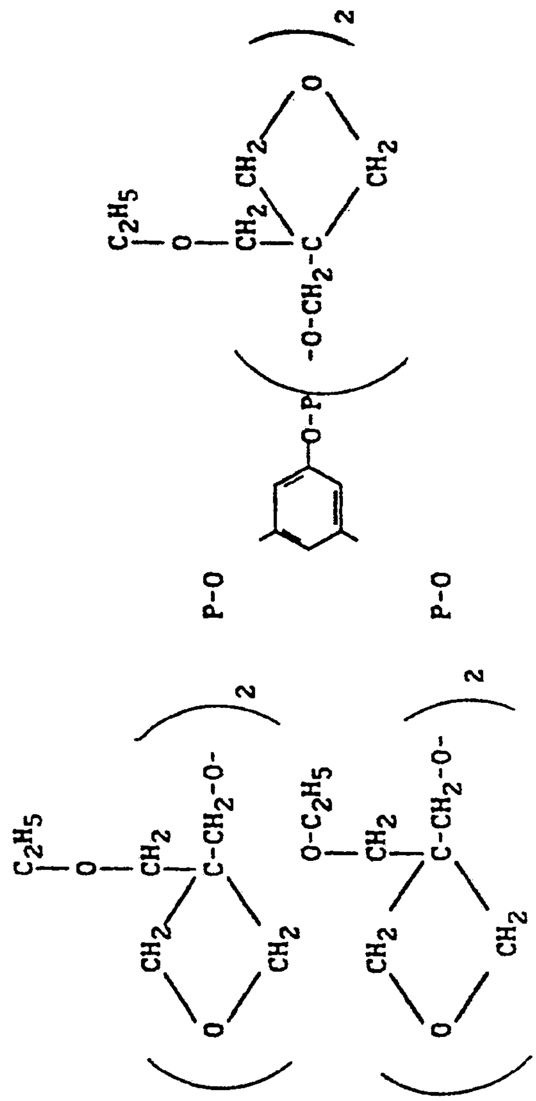

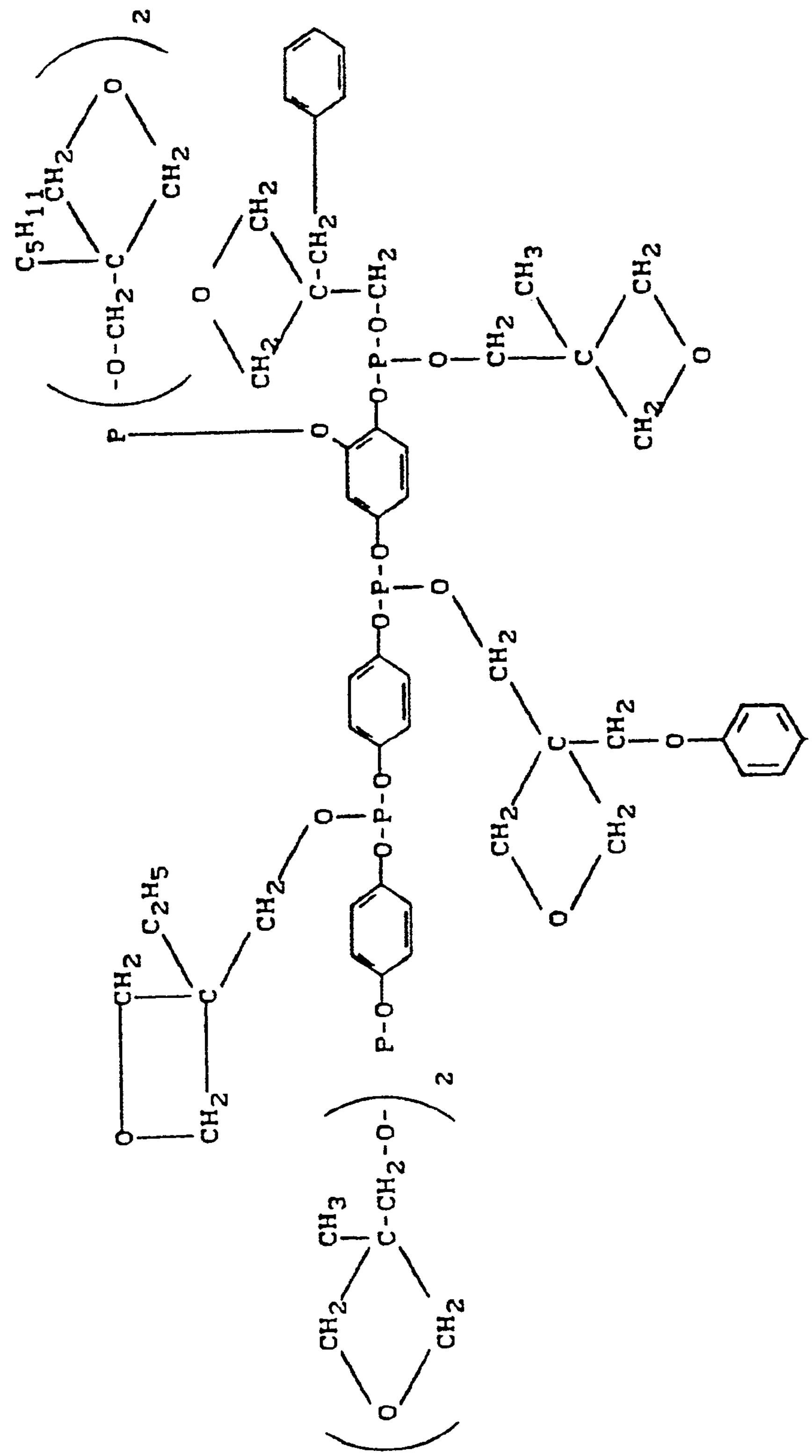

In den vorstehend genannten, erfindungsgemäß verwendbaren, oxetangruppenhaltigen Phosphorigsäureestern mit oxetangruppenfreien Polyalkoholen und/oder oxetangruppenfreien Polyhydroxyarylen können neben oxetangruppenhaltigen Monoalkoholen auch ocetangruppenfreie Monoalkohole und/oder oxetangruppenfreie Monohydroxyaryle mit eingebaut werden.

Erfindungsgemäß verwendbare Phosphorigsäureester sind schließlich auch solche aus oxetangruppenhaltigen Monoalkoholen und oxetangruppenfreie Monoalkohole und/oder oxetangruppenfreie Monohydroxyaryle und/oder oxetangruppenfreie Polyalkohole und/oder oxetangruppenfreie Polyhydroxyaryle mit eingebaut sein können.

Die erfindungsgemäß verwendbaren oxetangruppenhaltigen Phosphorigsäureester können allein oder im Gemisch zu mehreren eingesetzt werden.

Ein ganz besonders bevorzugt verwendbarer Phosphorigsäureester ist daß Tris-(2,2-dimethylenoxid-butyl)-phosphit.

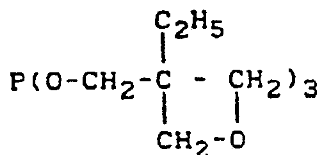

Man erhält die oxetangruppenhaltigen Phosphorigsäure-ester der Formeln (I), (IIIa) und (IIIb) in bekannter Weise durch Umesterung der entspreshenden oxetangruppenhaltigen Alkohole oder Phenole mit Trialkyl oder Trialkylphosphiten oder durch Umsatz von Phosphortrichlorid mit den entsprechenden Alkoholen oder Phenolen, gegebenenfalls in Mischung mit anderen ocetangruppenfreien Alkoholen oder Phenolen, in Gegenwart säurebindender Mittel beispielsweise gemäß US-P 3 209 013.

Oxetangruppenhaltige Phosphite der in Rede stehenden Art sind auch in den deutschen Patenten 2 140 207 (Le A 13 917) und 2 255 639 (Le A 14 709) beschrieben. Sie dienen dort zur Stabilisierung von thermoplastischen, aromatischen Polycarbonaten.

Die als Veresterungskatalysatoren wirksamen oxetangruppenhaltigen, neutralen Phosphite können in Mengen von 0,01 bis 10 Gew.-%, vorzugsweise von 0,1 bis 5 Gewichtsprozent, bezogen auf die Gewichtssumme aus Carbonsäure und Alkohol zugesetzt werden. Die Veresterungskatelysatoren können in verschiedener Form dem Reaktionsgemisch zugefügt werden. Man kann sie flüssig oder fest dem Reaktionsgemisch zugeben, ebenso in Lösung in einer der Ausgengsprodukte vorlegen, oder man dosiert die Veresterungskatelysatoren während der Reaktion zu. Vorzugweise legt man die Veresterungskatalysatoren zusammmen mit den Ausgangsprodukten vor.

Gegenstand der vorliegenden Erfindung ist somit außerdem ein Verfahren zur Herstellung von Carbonsäureestern aus Carbonsäuren und Alkoholen nach den bekennten Verfahren der Schmelzkondensation, der azeotropen Veresterung oder der extraktiven Veresterung, das dadurch gekennzeichnet ist, daß man als Veresterungskatalysatoren oxetangruppenhaltige, neutrale Phosphite in Mengen von 0,01 bis 5 Gewichtsprozent, bezogen auf die Gewichtssumme aus Carbonsäure und Alkohol, einsetzt.

Das Verfahren der Schmelzkondensation ist charakterisiert, dadurch, daß die Ausgangskomponenten in flüssigem oder geschmolzenem Zustand bis zur Reaktionstemperatur erhitzt werden. Dabei spaltet sich Wasser, gegebenenfalls unter Durchleiten von Intergas ab. Die Veresterung ist beendet, wenn kein Reaktionswasser mehr entsteht.

Das Verfahren der azeotropen Veresterung ist charakterisiert dadurch, daß man die Veresterung in einem Lösungsmittel ausführt, welches mit Wasser ein Azeotrop bildet, Das entstehende Reaktionswasser wird dann durch azeotrope Destillation aus dem Reaktionsgemisch entfernt.

Das Verfahren der extraktiven Veresterung ist charakterisiert dadurch, daß der gebildete Ester von einem Lösungsmittel aus dem Reaktionsgemisch herausgelöst wird. Das Lösungsmittel selbst darf nur wenig Wasser lösen.

(Bezüglich detaillierter Beschreibung dieser drei Verfahren, siehe "Organikum", 12. Auflage, NEB, Deutscher Verlag der Wissenschaften, Berlin 1973, Seiten 441 ff. sowie Houben-Weyl, "Methoden der Organischen Chemie, Sauerstoffverbindungen III, Band VIII, 1952, Seiten 516 bis 526 loc. cit.)

Geeignete Carbonsäuren, die sich nach dem erfindungsgemäßen Verfahren verestern lassen, sind Solche der Formel (IV)

$$R^6 (CO_2H)_n \quad (IV),$$

worin n eine ganze Zahl von 1 bis 6 ist und R der n-bindige Rest von linearen oder verzweigten Alkanen, die Etherbrücken enthalten können, Cycloalkanen, Arylalkanen oder Arenen ist, der zusätzlich durch Fluor substituiert sein kann. R kann auch Wasserstoff sein. Bevorzugte C-Reste $R^6$ sind $C_1$ bis $C_{30}$ enthaltende Ketten oder Ringe, aliphatischer oder aromatischer Struktur.

Geeignete Säuren der Formel (IV) sind beispielsweise Ameisensäure, Essigsäure, Propionsäure, Buttersäure, Isobuttersäure, Pentansäure, 2,2-Dimethylpropansäure, 2-Ethylhexansäure, Kokosfettsäure, Laurinsäure, Myristinsäure, Palmitinsäure, Stearinsäure, Eikosansäure, Behensäure, Lignocerinsäure, Sebacinsäure, Undecandisäure, Dodecandisäure, Brassylsäure, Thapsisäure, Butantetracarbonsäure, Naphthensäure, Hexahydrobenzoesäure, Benzoesäure, Phenylessigsäure, 4-tert.-Butylbenzoesäure, Hexahydroterephthalsäure, Phthalsäure, Cyclopentantetracarbonsäure, 4-Phenylphthalsäure, Diglykolsäure, Ethylglykolsäure, Trifluoressigsäure, Perfluorbenzoesäure, 3,4-Bis-(trifluormethyl)benzoesäure, p-Trifluormethylphenylessigsäure, Nonadecafluordecansäure, Octafluordipinsäure, 2H,2H-Heptadecafluordecansäure, 3H, 3H-Hexafluoradipinsäure, Perfluorglutarsäure und Perfluoroctansäure.

Geeignete Alkohole, die sich nach dem erfindungsgemäßen Verfahren verestern lassen, sind solche der formel (V)

$$(R^7)_n\text{-}C(CH_2OH)_{4-n}$$

worin n Null oder 1 oder 2 oder 3 ist und $R^7$ Wasserstoff, lineares oder verzweigtes Alkyl, Cycloalkyl, Alkoxy, Aralkyl oder Aryl sein kann, wobei im falle von n = 2 oder = 3, $R^7$ gleich oder verschieden sein kann.

Die Reste $R^7$ können außerdem durch fluor substituiert sein.

Bevorzugte C-Reste $R^7$ sind $C_1$ bis $C_{30}$ enthaltende Ketten oder Ringe aliphatischer oder aromatischer Struktur.

Geeignete Alkohole der Formel (V) sind Pentaerythrit, Glycerin, Trimethylolethan, Trimethylolpropan, Trimethylolhexan, Trimethyloloctedecan, 1,3-Propandiol, Neopentylglykol, 2-Methyl-2-propyl-1,3-propandiol, 2,2-Diethyl-1,3-propandiol, 1,4-Cyclohexandimethylol, Bismethylol-tricyclodecan, 1,1-Cyclohexandimethylol, Ethanol, Propanol, Neopentylalkohol, 2-Ethyl-1-butanol, Trimethyl-1-hxanol, Stearylalkohol, 2-Methoxyethanol, 2-Propoxyethanol, 2-(2-Butoxyethoxy)-ethanol, 2,2,2-Trifluorethanol, 1,3-Difluor-4-methylolbenzol, 1-Trifluormethyl-4-methylolbenzol, 1,2,4,5-Tetrafluor-3-trifluormethyl-6-methylolbenzol, 1,1,1-Trifluor-nonan-9-ol, 1H,1H,1H-2,2,3,3,4,4-Hexafluor-1,5-pentadiol, Pentafluorbenzylalkohol.

Weiterhin kommen auch Alkohole mit 4 bis 6 OH-Gruppen für das erfindungsgemäße Verfahren in Betracht, wie z.B. Arabit, Adonit, Mannit, Dulcit, Xylit, Sorbit.

In einer allgemeinen Ausführungsform legt man die Ausgangskomponenten zusammen oder getrennt nacheinander in festem, geschmolzenem oder in einem geeigneten Lösungsmittel gelöstem Zustand vor. Das Molverhältnis von $CO_2$-Gruppen zu –OH-Gruppen kann 0,5-2, vorzugsweise 0,8-1,2, betragen. Die Veresterung kann in einer Inertgasatmosphäre bei 170-280°C, vorzugsweise bei 200-250°C oder in einem Lösungsmittel bei geeigneter Temperatur erfolgen. Die Veresterung ist beendet, wenn die erforderliche Menge Wasser abgeschieden oder eine Säurezahl oder Hydroxylzahl von $<$ 10 mg KOH/g erreicht worden ist.

Eine spezielle Ausführungsform wird in den Beispielen beschrieben. Es ist auch möglich, nach Herstellung die erhaltenen Ester durch fraktionierte Kristallisation oder Destillation zu reinigen.

Ein wesentlicher und überraschender Vorteil des erfindungsgemäßen Verfahrens ist die schnellere Veresterung bei gleichzeitiger niedriger OHZ und SZ des dabei gebildeten Produktes, wie in den Beispielen dargelegt ist.

Die erhaltenen Carbonsäureester sind im allgemeinen bekannte Verbindungen. Sie sind thermisch brauchbar teils als Lösungsmittel oder als Zwischenprodukte für organische Synthesen in bekannter Weise.

Ein Teil der nach dem erfindungsgemäßen Verfahren erhältlichen Carbonsäureestern ist besonders gut geeignet als Entformungsmittel für thermoplastische aromatische Polycarbonate gemäß den deutechen Offenlegungsschriften DEOS 2 0654 095 (Le A 13 461), DE-OS 2 220 185 (Le A 14 329), DE-OS 2 507 748 (Le A 16 284) und DE-OS 2 729 485.

Die Wirksamkeit des erfindungsgemäßen Verfahrens sei an folgenden Beispielen und Vergleichsversuchen demonstriert.

Allgemeine Vorschrift

1,1 Mol Stearinsäure, 0,25 Mol Pentaerythrit, 2,5 g Katalysator werden in einer 2 1-Dreihalsglasapparatur mit absteigendem Kühler unter $N_2$ auf 200°C aufgeschmolzen und unter Kühren bei dieser Temperatur gehalten bis sich 18 ml $H_2O$ (1 Mol) abgeschieden haben. Die Zeit bis zu diesem Reaktionsende und die dabei erhaltenen Säurezahlen und Hydroxylzahlen sind in der nachfolgenden Tabelle in Abhängigkeit von verschiedenen Katalysatoren aufgelistet. Säurezahl und Hydroxylzahl werden gemäß DIN 53 402 bzw. nach P.H. Selden, "Glasfaserverstärkte Kunststoffe", S. 444 (1967) bestimmt.

| Nr. | Katalysator | Zeit bis zur Abspaltung von 1 Mol $H_2O$ (h) | SZ mg KOH/g | OHZ mg KOH/g | Farbe |
|---|---|---|---|---|---|
| 1 | 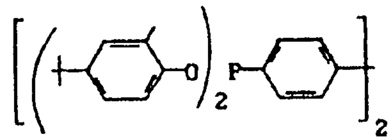 | 8 | 37 | 73 | dunkelbraun |
| 2 | 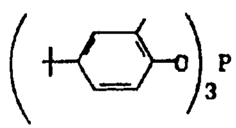 | 8 | 27 | 37 | " |
| 3 | $H_3PO_3$ | 32 | 27 | 4 | " |
| 4 | $(HO-CH_2)_3P$ | 24 | 23 | 15 | " |
| 5 | $(C_4H_9O)_4Ti$ | 40 | 20 | 6 | " |
| 6 | 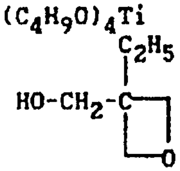 | 64 | 14 | 15 | gelb |
| 7 | - | 24 | 19 | 21 | gelb |
| 8 | 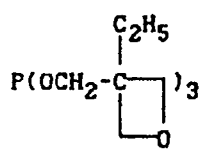 | 6 | 14 | 6,8 | weiß |
| 9 | 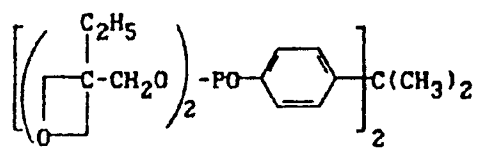 | 7 | 11 | 6 | weiß |

## Ansprüche

1. Verwendung von oxetangruppenhaltigen neutralen Phosphiten zur Herstellung von Carbonsäureestern aus Carbonsäuren und Alkoholen.

2. Verwendung der Phosphite gemäß Anspruch 1 in Mengen von 0,01 bis 10 Gew.-%, bezogen auf die Gewichtssumme aus Carbonsäure und Alkohol.

3. Verwendung der Phosphite gemäß Anspruch 1 in Mengen von 0,1 bis 5 Gew.-%, bezogen auf die Gewichtssumme aus Carbonsäure und Alkohol.

4. Verfahren zur Herstellung von Carbonsäureestern aus Carbonsäuren und Alkoholen nach den bekannten Verfahren der Schmelzkondensation, der azeotropen Veresterung oder der extraktiven Veresterung, dadurch gekennzeichnet, daß man als Veresterungskatalysatoren oxetangruppenhaltige, neutrale Phosphite in Mengen von 0,01 bis 10 Gewichtsprozent, bezogen auf die Gewichtssumme aus Carbonsäure und Alkohol, einsetzt.

5. Verfahren gemäß Anspruch 4, dadurch gekennzeichnet, daß man die Phosphite in Mengen von 0,1 bis 5 Gew.-% einsetzt.

## Claims

1. The use of neutral phosphites containing oxetane groups for the preparation of carboxylic acid esters from carboxylic acids and alcohols.

2. The use of the phosphites according to Claim 1 in quantities of from 0.01 to 10% by weight, based on the sum of the weights of carboxylic acid and alcohol.

3. The use of the phosphites according to Claim 1 in quantities of from 0.1 to 5% by weight, based on the sum of the weights of carboxylic acid and alcohol.

4. A process for the preparation of carboxylic acid esters from carboxylic acids and alcohols by the known processes of solvent-free condensation, azeotropic esterification or extractive esterification, characterised in that the esterification catalysts used are oxetane group-containing neutral phosphites in quantities of from 0.01 to 10% by weight, based on the sum of the weights of carboxylic acid and alcohol.

5. A process according to Claim 4, characterised in that the phosphites are used in quantities of from 0.1 to 5% by weight.

## Revendications

1. Utilisation de phosphites neutres portant des groupes oxétanne pour la production d'esters carboxyliques d'acides carboxyliques et d'alcools.

2. Utilisation des phosphites suivant la revendication 1 en quantités de 0,01 à 10% en poids par rapport à la somme des poids d'acide carboxylique et d'alcool.

3. Utilisation des phosphites suivant la revendication 1 en quantités de 0,1 à 5% en poids par rapport à la somme des poids d'acide carboxylique et d'alcool.

4. Procédé de production d'esters d'acides carboxyliques à partir d'acides carboxyliques et d'alcools par les procédés connus de condensation à l'état fondu, d'estérification azéotropique ou d'estérification par extraction, caractérisé en ce qu'on utilise comme catalyseurs d'estérification des phosphites neutres porteurs de groupes oxétanne en quantités de 0,01 à 10% en poids sur la base de la somme des poids d'acide carboxylique et d'alcool.

5. Procédé suivant la revendication 4, caractérisé en ce qu'on utilise les phosphites en quantités de 0,1 à 5% en poids.